Europäisches Patentamt

European Patent Office

Office européen des brevets

(11) Publication number: **0 174 180**
**B1**

(12) EUROPEAN PATENT SPECIFICATION

(45) Date of publication of patent specification: 23.08.89

(51) Int. Cl.⁴: **A 61 L 17/00**

(21) Application number: 85306241.2

(22) Date of filing: 03.09.85

(54) Coated monofilament sutures.

(30) Priority: 04.09.84 US 646534

(43) Date of publication of application:
12.03.86 Bulletin 86/11

(45) Publication of the grant of the patent:
23.08.89 Bulletin 89/34

(84) Designated Contracting States:
DE FR GB IT SE

(56) References cited:
US-A-3 942 532
US-A-4 105 034

(73) Proprietor: ETHICON INC.
U.S. Route 22
Somerville New Jersey 08876 (US)

(72) Inventor: Kafrawy, Adel
20 Hickory Trail
Flemington New Jersey 08822 (US)
Inventor: Hunter, Alastair Wilson
516 Spring Valley Drive
Bridgewater New Jersey 08876 (US)

(74) Representative: Jones, Alan John et al
CARPMAELS & RANSFORD 43 Bloomsbury
Square
London, WC1A 2RA (GB)

## Description

The invention relates to coated monofilament sutures that have improved tiedown and package delivery properties.

### Background of the invention

A variety of materials are used to alter the surface characteristics of surgical sutures. Among the properties that are often sought to be altered by coatings on sutures are tiedown properties, lubricity as it pertains to ease of passage through tissue, ease of package dispensing, and minimizing fibrillation of monofilament suture surfaces during dispensing.

In most cases, lubricating coatings are used on multifilament braided sutures. Up until now, comparatively little research effort has been expended on efforts to find lubricating coatings for monofilament sutures because it was felt that such coatings were not needed, and were also thought to have poor adhesion to monofilament sutures. It was also thought that coating monofilament sutures could compromise knot security, thereby requiring additional throws to secure the knot. This invention is based upon the discovery that a particular low molecular weight polyester can be used as a coating on monofilement sutures, with the result that one or more properties of the sutures thus coated are improved.

### Brief summary of the invention

The invention provides monofilament surgical filaments having a thin coating of a low molecular weight aliphatic polyester with a number average molecular weight of from 1,000 to 15,000 and having an acid component containing at least four carbon atoms. The surgical filaments of the invention exhibit improved tie-down properties, and often other properties such as package delivery characteristics are also improved.

### The prior art

Hunter et al., U.S. Patent No. 3,942,532, disclose the use of low molecular weight at aliphatic polyesters, including polybutylene adipate, as lubricating coatings for braided multifilament sutures to improve their tie-down properties.

Other patents that disclose the use of various materials as lubricating coatings on sutures include the following:

Schmitt, et al., U.S. Patent No. 3,982,543
Mattei, U.S. Patent No. 4,027,676
Casey, et al., U.S. Patent No. 4,080,969
Casey, et al., U.S. Patent No. 4,095,600
Shalaby, et al., U.S. Patent No. 4,105,034
Mattei, U.S. Patent No. 4,185,637
Mattei, U.S. Patent No. 4,201,216
Usher et al., British Patent No. 1,258,688

The disclosures of these patents are predominantly of the use of various polymeric materials as coatings on braided multifilament surgical filaments such as sutures. However, an occasional mention is made of the possibility of coating a monofilament suture as well. See, for example, Casey, et al., No. 4,080,969, col. 8, lines 24—25.

### Brief description of the drawings

Figure 1 is a diagrammatic representation of an INSTRON Tester and shows two suture strands in position for testing tie-down roughness;

Figure 2 is an enlarged perspective view of the single throw knot illustrated in Fig. 1;

Figure 3 is a reproduction of the tracing of an oscillographic recorder; and

Figure 4 is a schematic representation of the set up for the knot security test.

### Detailed description of the invention

The polyesters used in this invention as lubricating coatings on surgical monofilaments such as sutures and ligatures are aliphatic polyesters having a number average molecular weight of from 1000 to 15,000, and preferably from 2000 to 3000, as determined by vapor phase osmometry.

The polyesters used as lubricating coatings in this invention are those derived from alkanediols and aliphatic dibasic acids wherein the alkanediols have at least two carbon atoms and wherein the aliphatic dibasic acids have at least four carbon atoms. It is preferred to employ polyesters of polymethylene glycols and straight chain alpha, omega-alkanedioic acids. Illustrative diols which can be used to produce the polyester include ethylene glycol, 1,2- and 1,3-propylene glycol, 1,4-butanediol, pentane diol, hexamethylene glycol, octamethylene glycol, and the like. The most preferred glycol is 1,4-butanediol. Illustrative acids are succinic, glutaric, adipic, pimelic, suberic, azelaic, sebacic, or mixtures thereof. The preferred acid is adipic. Poly(butylene adipate) is the preferred polyester.

The polyester coating of the invention is applied to the surface of a surgical monofilament such as a suture or ligature. The monofilament may be made from polypropylene, homopolymers and copolymers of p-dioxanone, poly[tetramethylene terephthalate-co-(2-alkenyl- or 2-alkyl)succinate] (see U.S. Patent No. 4,388,926), ethylene-propylene copolymer, vinylidene fluoridehexafluoropropylene copolymer, nylon, and polyethylene terephthalate. (Polyethylene terephthalate is used as a monofilament suture almost exclusively in very fine sizes, e.g. U.S.P. 9/0 to 11/0, in micro-surgery.)

The coating is preferably applied to the monofilament by contacting the monofilament with a dilute solution or suspension of the polyester in a suitable vehicle such as toluene, other aromatic hydrocarbons, esters, chlorinated hydrocarbons, mixtures thereof, and the like, followed by drying the coated monofilament by any convenient procedure to remove the vehicle. In laboratory batch scale operations, the individual lengths of monofilament can be dipped into the polyester solution or suspension for a few seconds (e.g., 1 to 5 seconds are satisfactory), followed by blotting the coated monofilaments, centrifugation, and/or air drying of the coated monofilaments. On a production scale operation, continuous dipping followed by passage through a force air oven kept at moderate temperatures [e.g., from about 150° to about 175°F (66 to 79°C)] can be employed.

The concentration of the polyester in the vehicle is kept rather low, for instance, from about 1 to about 6 weight per cent, based on weight of polyester plus vehicle. This results in add-on levels of from 0.03 to 3 weight percent, based on weight of monofilament.

The coated monofilaments can be sterilized by the usual methods that are used for the uncoated monofilaments. Thus, ethylene oxide or exposure to gamma radiation can be used to sterilize the coated monofilaments. When the monofilaments are used in the form of sutures (the preferred aspect of this invention), they may be attached to a surgical needle by the usual methods.

The examples below illustrate the invention.

Experimental procedure

Four different types of monofilament sutures were coated with poly(1,4-butylene adipate) having a number average molecular weight of 2000—2400, by the following procedure:

Three different concentrations of polyester in toluene were used, 1.02% w/w, 2.10% w/w, and 3.00% w/w. The sutures in annealed cut bundle form were dip coated for 30 seconds in the coating bath and then immediately transferred to a laboratory size centrifuge where the sutures were centrifuged free of excess coating for 5 minutes at a speed of 2500—4000 RPM. The sutures were then hung from a line under a hood for 4 hours to flash off the toluene solvent.

A complete physical evaluation of the sutures was done where there was sufficient material for testing. The physical tests included diameter, dry and wet knot tensile, dry straight tensile, elongation %, Youngs modulus, dry and wet tiedown roughness, and dry and wet knot security. Also included in the tests were visual appearance evaluation, subjective tactile smoothness evalution, and microscopic appearance evaluation.

The coated sutures were also analyzed for add-on of the polyester coating and for package delivery characteristics.

The four types of monofilament sutures that were evaluated were made from the following polymers:

Example 1

A copolyster containing 72±2 weight percent of polymerized tetramethylene terephthalate units and 28±2 weight percent of polymerized tetramethylene 2-octadecenylsuccinate units.

Example 2

A copolymer of ethylene and propylene containing 1.6±0.2 weight percent polymerized ethylene. The copolymer had a melt flow by ASTM D-1238, Condition L, of 2.

Example 3

A random copolymer containing 90±2 weight percent of polymerized vinylidene fluoride and 10±2 weight percent of polymerized hexafluoropropylene, having a melting point by ASTM D-3418 of 160°C. and an apparent melt viscosity of 22,000 poise at 200°C. and 100 sec$^{-1}$.

Example 4

Isotactic polypropylene.

The procedures used for analysis were the following:

Tie-down roughness measurements are made on a Table-Model INSTRON Tensile Tester using a Type B tension cell, full-scale range 100 to 2,000 grams. The INSTRON instrument is manufactured by the Instron Corporation of Canton, Massachusetts. A high-speed SANBORN Oscillographic Recorder (Model 7702A, manufactured by Hewlett-Packard, Waltham, Massachusetts) is substituted for the standard INSTRON Recorder which would be too slow to follow the rapid changes in force that result as the sutures under test slide against each other. A high-gain DC Amplifier (Hewlett-Packard Model 8803A, manufactured by Hewlett-Packard, Waltham Division, Waltham Massachusetts) is used to interface this recorder with the INSTRON Transducer and a low-voltage DC power supply is provided to excite the transducer. The measurements are made in an air-conditions laboratory at 72°F (22°C) and 50 percent relative humidity. To hold the specimen suture strands, a line contact jaw is used. The INSTRON machine is operated at a cross-head speed of 50 inches per minute (127 cm/min) and the chart speed of the oscillographic recorder is 20 millimeters per second.

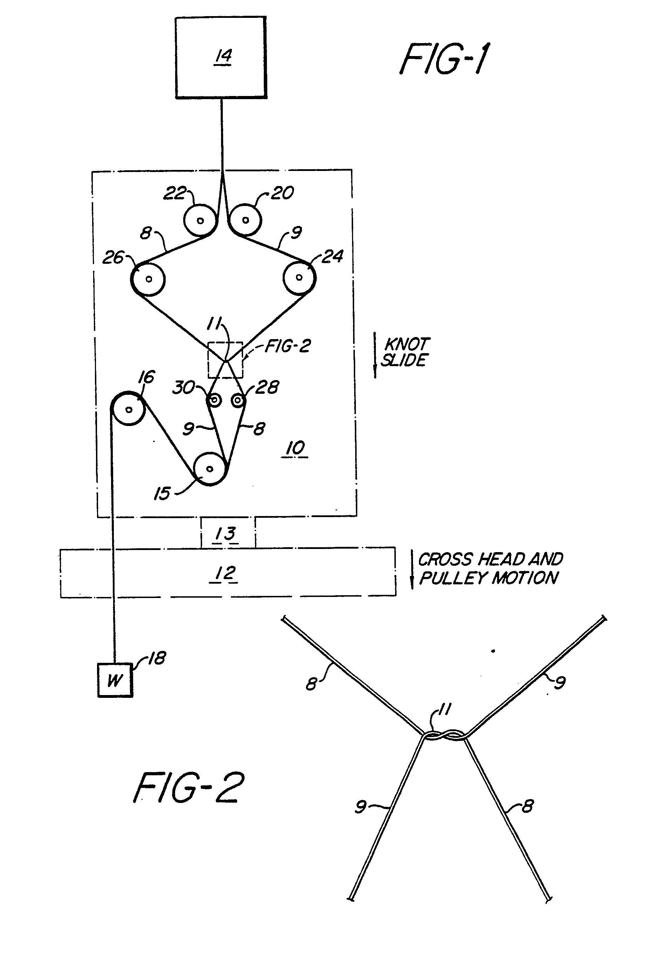

Subjective tests for "tie-down" involve the suture configuration 11 shown in Fig. 2 (a single throw knot). The same configuration is produced by a pulley arrangement that is supported by a steel plate 10 shown in Fig. 1. The steel plate is attached to the cross-head 12 of the INSTRON Tester by a connector 13.

To perform tie-down roughness measurements, two strands 8 and 9 of the same suture are attached at one end to the fixed B cell transducer 14 of an INSTRON Tester. The sutures are threaded through the pulley arrangement as shown in Figs. 1 and 2. The other end of the suture strands are brought together, passed around the pulleys 15 and 16, and attached together to a weight (W) 18 which provides tension similar to that applied in a subjective test. As the Instron crosshead 12 moves down, and with it the attached plate 10 and pulleys 15, 16, 20, 22, 24, 26, 28, 30, the knot 11 slides along the suture at the same speed as the crosshead 12. The total downward force (slide force) exerted by the upper end of the suture on the transducer 14 is equal to the tension weight (w) plus the force required to slide the knot 11. As the knot 11 moves down the suture, the sliding force varies rapidly between limits. These force variations are the roughness felt in subjective tie-down tests and the amplitude of the variations is a measure of the roughness.

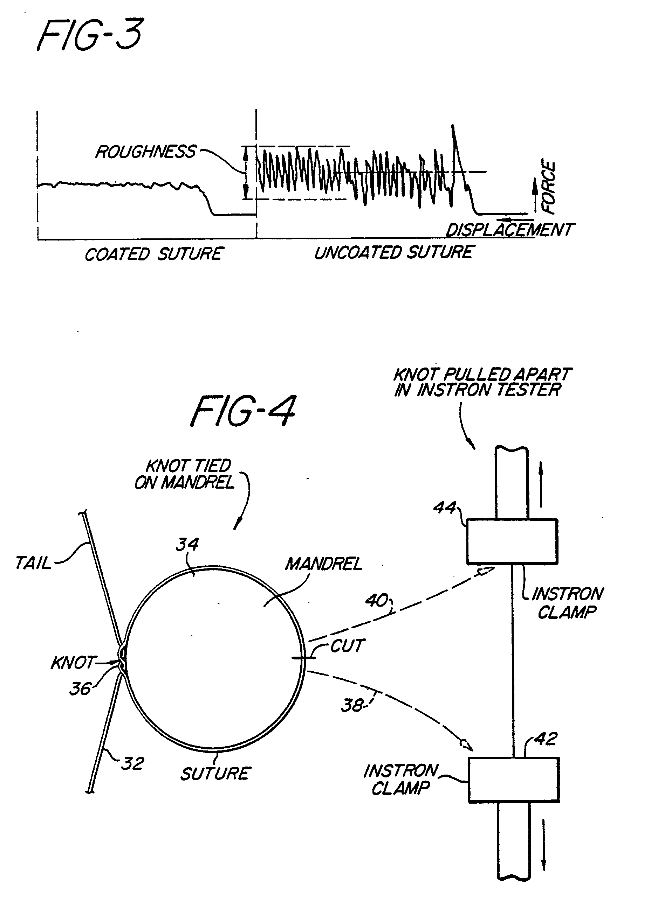

Figure 3 shows typical recorder traces for a suture before and after coating to improve tie-down roughness performance. The roughness values (i.e., amplitude of the variations in sliding force) are measured along the ordinate and throughout the specification and examples are recorded in grams (roughness). When relatively smooth samples are compared, the amplitude of the oscillographic recorder can be increased by a factor of 20.

Knot security is determined by the following procedure:

Dry test method

Referring to Figure 4, a loop of suture material 32 is formed around a three-inch mandrel 34 and a knot 36 with varying number of squared throws tied by hand. The loop is cut as illustrated in Figure 4, and the cut ends 38, 40 are clamped in the jaws 42, 44 of an Instron Tensile Tester. The knot 36 is pulled apart with a crosshead speed of 2 inches/minute (5.08 cm/min) and will either slip or break. The lowest force at which slipping occurs is measured or, if no slipping occurs, the force required for breaking. Twenty knots are pulled apart for the force required for each size suture and for each number of throws.

A typical determination is made by tying 20 knots with two squared throws and pulling them apart. Assuming they all slip, 20 knots with three squared throws are tied and pulled apart. This process is continued with increasing throws until all 20 knots break cleanly without slipping. The forces are then computed separately for each throw to determine the average force and standard deviation at which absolute knot security can be obtained.


Wet test method

Knots are tied as described for dry testing. Groups are labelled according to the number of throws. The entire piece of suture containing the knot is then soaked in reclaimed human plasma for approximately 24 hours at 38°F (3°C) or in normal saline (Sodium Chloride, 0.9%) for approximately 24 hours at room temperature in glass containers and then pulled apart on an Instron Tester. Except for soaking, the procedure is the same as for dry testing.

A standard U.S.P. gauge of the dead weight type was used to measure suture diameters.

The procedure for determining the knot strength of the sutures was as follows:

Place a six-inch piece of rubber tubing 1/8" (3.2 mm) inside diameter and 1/16" (1.5 mm) wall thickness into the slot of a knot tying stand. Bring the two cut ends of the suture material up and over the rubber tubing holding the left end of the strand in the left hand and the right end of the strand in the right hand.

Cross the left end of the strand over the right end of the strand and loop twice (left over right). Bring the loop down to the rubber tubing as freely as possible so as not to "saw" down on the strand. Pull the ends taut; not strangulating tight or loose on rubber tubing.

Secondly, cross the right end of the strand over the left end of the strand (right over left), making one loop and bring the loop down directly on top of the first tie-down so that a single knot is superimposed upon a compound knot. Again, care should be taken not to saw down on the strand and that tightening is taut, but not tight.

Remove the rubber tubing from the knot tying stand without placing fingers on the knot.

Place on free end of the suture material into the right clamp of an IP-4 Machine and secure the clamp. The strand is to emerge from the drum or round side of the clamps.

Return to specimen and complete securing into the clamp by bringing suture material under the right drum and straight across to the left clamp with the knot approximately midway between the clamps. Holding the material firmly, bring the left end up and around the drum into the clamp and secure the clamp. The strand is not loose but somewhat taut between the clamps.

Set the pen on the pen line on the test chart and release the pen against the chart for action.

The Incline Plane Testing Machine known as the IP-4 is a motor driven tensile strength testing machine using the principle of the constant specimen-rate-of-load, having suitable clamps for holding the specimen firmly.

Check for level of machine with compensator prior to commencing with tests at the start of each shift. At the level position the pen is on zero line. On the IP-4 during operating condition, the pen is set on pen line below the zero line.

The distance between the clamps was 7 inches (17.8 cm). An Instron tensile tester was used to measure straight tensile strength, elongation at break, and Young's modulus. The Instron settings were as follows:

TABLE I

For Examples 1 and 3:

| | Crosshead speed in/min (cm/min) | Chart speed in/min (cm/min) | Gauge length in. (cm) | Scale load lbs (N) |
|---|---|---|---|---|
| Straight tensile and elongation | 5 (12.7) | 10 (25.4) | 5 (12.7) | 20 (89) |
| Young's modulus | 5 (12.7) | 10 (25.4) | 5 (12.7) | 10 (44.5) |

TABLE II

For Examples 2 and 4:

| | Crosshead speed in/min (cm/min) | Chart speed in/min (cm/min) | Gauge length in. (cm) | Scale load lbs (N) |
|---|---|---|---|---|
| Straight tensile and elongation | 5 (12.7) | 20 (50.8) | 10 (25.4) | 5 (22.2) |
| Youngs' modulus | 1 (2.5) | 5 (12.7) | 1 (2.5) | 10 (44.5) |

Table III, below, displays the add-on levels of the sutures for the three different concentrations of polybutylene adipate in toluene:

TABLE III

| Sample | USP suture size | Concentration of polyester in toluene, % | Add-on, % |
|---|---|---|---|
| Example 1-A | 0 | 1.02 | 0.033[1] |
| Example 1-B | 0 | 2.10 | 0.260 |
| Example 1-C | 0 | 3.00 | 0.35 |
| Control 1 | 0 | — | — |
| Example 2-A | 2/0 | 1.02 | 0.11[2] |
| Example 2-B | 2/0 | 2.10 | 0.14 |
| Example 2-C | 2/0 | 3.00 | 0.17 |
| Control 2 | 2/0 | — | — |
| Example 3-A | 2/0 | 1.02 | 0.03[2] |
| Example 3-B | 2/0 | 2.10 | 0.06 |
| Example 3-C | 2/0 | 3.00 | 0.07 |
| Control 3 | 2/0 | — | — |
| Example 4-A | 2/0 | 1.02 | 0.07[2] |
| Example 4-B | 2/0 | 2.10 | 0.17 |
| Example 4-C | 2/0 | 3.00 | 0.24 |
| Control 4 | 2/0 | — | — |

[1] By gravimetric analysis.

[2] By gas chromatographic analysis of the polyester hydrolyzate 1,4-butanediol and compared to standard solutions.

The wet and dry tiedown roughness data are displayed below in Table IV. Each number given is the average of four samples. The standard deviations are given in parentheses. All weights are in grams.

# EP 0 174 180 B1

## TABLE IV

| Sample No. | Weight (W) | Dry tie-down | | Wet tie-down | |
|---|---|---|---|---|---|
| | | Roughness | Slide force | Roughness | Slide force |
| Example 1-A | 900 | 329 (140) | 1168 (126) | 598 (117) | 962 (84) |
| Example 1-B | 900 | 305 (96) | 1156 (114) | 203 (31) | 1135 (85) |
| Example 1-C | 900 | 215 (92) | 1064 (106) | 191 (44) | 1088 (51) |
| Control 1 | 900 | 466 (179) | 1273 (104) | 843 (187) | 971 (106) |
| Example 2-A | 700 | 412 (185) | 1055 (20) | 526 (55) | 992 (60) |
| Example 2-B | 700 | 257 (53) | 1007 (54) | 400 (174) | 1091 (55) |
| Example 2-C | 700 | 275 (106) | 1040 (57) | 460 (106) | 995 (49) |
| Control 2 | 700 | 466 (60) | 998 (12) | 526 (101) | 992 (45) |
| Example 3-A | 700 | 119.5(0) | 1064 (20) | 114 (23) | 1073 (6) |
| Example 3-B | 700 | 108 (14) | 1040 (10) | 173 (140) | 1168 (241) |
| Example 3-C | 700 | 126 (12) | 1037 (40) | 114 (12) | 1049 (11) |
| Control 3 | 700 | 269 (126) | 1127 (40) | 490 (185) | 992 (106) |
| Example 4-A | 700 | 221 (60) | 1061 (51) | 538 (405) | 1034 (86) |
| Example 4-B | 700 | 191 (70) | 980 (35) | 179 (4) | 1147 (48) |
| Example 4-C | 700 | 173 (127) | 1019 (48) | 878 (6) | 1067 (45) |
| Control 4 | 700 | 227 (124) | 998 (32) | 460 (315) | 1037 (41) |

In Table V, below, the data found for the knot security evaluations are displayed. The results are reported in pounds force [Newton equivalents shown in parentheses], with the standard deviations being given in paranetheses.

7

TABLE V

| Sample | 3 Throws Dry | | 3 Throws Wet | | 4 Throws Dry | | 4 Throws Wet | |
|---|---|---|---|---|---|---|---|---|
| Example 1-A | 4.6(0.4) | [20.5(1.8)] | 4.6(0.5) | [20.5(2.2)] | 6.45(0.3) | [28.7(1.3)] | 7.1(1) | [31.6(4.4)] |
| Example 1-B | 4.3(0.5) | [19.1(2.2)] | 4.9(0.7) | [21.8(3.1)] | 6.2(0.6) | [27.6(2.7)] | 7.3(0.5) | [32.5(2.2)] |
| Example 1-C | 4.4(0.4) | [19.6(1.8)] | 4.7(0.4) | [20.9(1.8)] | 6.8(0.6) | [30.2(2.7)] | 7.5(0.7) | [33.4(3.1)] |
| Control 1 | 5.2(0.7) | [23.1(3.1)] | 5.7(0.5) | [25.4(2.2)] | 6.75(0.77) | [30.0(3.4)] | 7.13(1) | [31.7(4.4)] |
| Example 2-A | 5.3(0.6) | [23.6(2.7)] | 5.6(0.8) | [24.9(3.6)] | 6(0.2) | [26.7(0.9)] | 6.5(0.3) | [28.9(1.3)] |
| Example 2-B | 5.7(0.2) | [25.4(0.9)] | 6(0.4) | [26.7(1.8)] | — | — | 6.4(0.3) | [28.5(1.3)] |
| Example 2-C | 5.3(0.5) | [23.6(2.2)] | 5.4(0.8) | [24.0(3.6)] | 6.1(0.2) | [27.1(0.9)] | 6.5(0.3) | [28.9(1.3)] |
| Control 2 | 5.6(0.6) | [24.9(2.7)] | 6.6(0.3) | [29.4(1.3)] | — | — | — | — |
| Example 4-A | 6.1(0.4) | [27.1(1.8)] | 6(0.4) | [26.7(1.8)] | — | — | 6.5(0.3) | [28.9(1.3)] |
| Example 4-B | 6(0.2) | [26.7(0.9)] | 6(0.3) | [26.7(1.3)] | — | — | — | — |
| Example 4-C | 5.8(0.5) | [25.8(2.2)] | 6(0.2) | [26.7(0.9)] | 6.1(0.3) | [27.1(1.3)] | — | — |
| Control 4 | 5.8(0.4) | [25.8(1.8)] | 6.3(0.3) | [26.7(1.3)] | — | — | — | — |

The measured diameters and the tensile data for the sutures are displayed below in Tables VI through IX:

TABLE VI
Poly(tetramethylene terephthalate co-2-octadecenylsuccinate)

| | Example 1 A | Example 1 B | Control 1 C | Control 1 |
|---|---|---|---|---|
| Diameter, mils[μm] | 14.8[376] (0.17)[(4.3)] | 14.7[373] (0.26)[(6.6)] | 14.8[376] (0.18)[(4.6)] | 14.8[376] (1.25)[(39.7)] |
| Knot pull, lbs[N] | 6.13[27.3] (0.6)[(2.7)] | 5.93[26.4] (0.99)[(4.4)] | 6.13[27.3] (0.27)[(1.2)] | 6.28[27.9] (0.62)[(2.8)] |
| Knot intrinsic, psi[MPa] | 35,700[246] | 35,090[242] | 35,530[245] | 36,640[253] |
| Straight pull, lbs[N] | 11.64[51.8] (0.45)[(2.0)] | 11.92[53.0] (0.15)[(0.6)] | 11.93[53.1] (0.28)[(1.2)] | 11.86[52.8] (0.16)[(0.7)] |
| Straight intrinsic, psi[MPa] | 67,780[467] | 70,590[487] | 69,130[477] | 69,160[477] |
| Elongation, % | 39 (2.6) | 40 (1.6) | 41 (2) | 39 (3) |
| Young's modulus, psi[MPa] | 110,800[764] | 107,000[738] | 106,500[734] | 103,000[710] |

(Standard deviations are in parentheses.)

8

TABLE VII
Ethylene-propylene copolymer

|  | Example 2 | | Control 2 | |
|---|---|---|---|---|
|  | A | B | C | |
| Diameter, mils[μm] | 12.7[323]<br>(0.42)[(11)] | 12.7[323]<br>(0.35)[(8.9)] | 12.8[325]<br>(0.37)[(9.4)] | 12.6[320]<br>(0.38)[(9.7)] |
| Knot pull, lbs[N] | 5.53[24.6]<br>(0.51)[(2.3)] | 5.03[22.4]<br>(0.54)[(2.4)] | 5.30[23.6]<br>(0.33)[(1.5)] | 4.83[21.5]<br>(0.53)[(2.35)] |
| Knot intrinsic, psi[MPa] | 43,400[299] | 39,700[274] | 41,300[285] | 38,650[266] |
| Straight pull, lbs[N] | 8.97[39.9]<br>(0.19)[(0.8)] | 8.90[39.6]<br>(0.33)[(1.5)] | 9.08[40.4]<br>(0.15)[(0.7)] | 8.75[38.9]<br>(0.53)[(2.4)] |
| Straight intrinsic, psi | 70,400[485] | 70,200[484] | 70,700[487] | 70,000[483] |
| Elongation, % | 75<br>(5) | 70<br>(9) | 71<br>(9) | 74<br>(10) |
| Young's modulus, psi[GPa] | 367,000[2.53] | 394,000[2.72] | 391,000[2.7] | 388,000[2.68] |

TABLE VIII
Vinylidene fluoride-hexafluoropropylene copolymer

|  | Example 3 | | Control 3 | |
|---|---|---|---|---|
|  | A | B | C | |
| Diameter, mils[μm] | 12.50[318]<br>(0.14)[(3.6)] | 12.47[317]<br>(0.11)[(2.8)] | 12.46[316]<br>(0.14)[(3.6)] | 12.55[319]<br>(0.13)[(3.3)] |
| Knot pull, lbs[N] | 5.58[24.8]<br>(0.34)[(1.5)] | 5.51[24.5]<br>(0.41)[(1.8)] | 5.57[24.8]<br>(0.28)[(1.2)] | 5.53[24.6]<br>(0.53)[(2.4)] |
| Knot intrinsic, psi[MPa] | 45,500[314] | 45,100[311] | 45,700[315] | 44,700[308] |
| Straight pull, lbs[N] | 9.59[42.6]<br>(0.19)[(0.8)] | 9.58[42.6]<br>(0.36)[(1.6)] | 9.64[42.9]<br>(0.29)[(1.3)] | 9.55[42.5]<br>(0.34)[(1.5)] |
| Straight intrinsic, psi[MPa] | 78,100[538] | 78,400[541] | 79,000[545] | 77,200[532] |
| Elongation, % | 36<br>(2) | 38<br>(1.8) | 36<br>(3) | 34<br>(2) |
| Young's modulus, psi[GPa] | 229,000[1.58] | 228,000[1.57] | 226,000[1.56] | 223,500[1.54] |

TABLE IX
Isotactic polypropylene

| | Example 4 | | Control 4 | |
| --- | --- | --- | --- | --- |
| | A | B | C | |
| Diameter, mils[μm] | 11.98[304] (0.68)[(17.2)] | 11.89[302] (0.65)[(16.5)] | 11.79[299] (0.56)[(14.2)] | 11.95[304] (0.63)[(16.0)] |
| Knot pull, lbs[N] | 5.81[25.8] (0.59)[(2.6)] | 5.53[24.6] (0.37)[(1.6)] | 5.70[25.4] (0.37)[(1.6)] | 5.69[25.3] (0.37)[(1.6)] |
| Knot intrinsic, psi[MPa] | 51,500[355] | 49,800[343] | 52,200[360] | 50,800[350] |
| Straight pull, lbs | 8.62[38.3] (0.08)[(0.35)] | 8.62[38.3] (0.04)[(0.2)] | 8.65[38.5] (0.1)[(0.44)] | 8.78[39.1] (0.08)[(0.35)] |
| Straight intrinsic, psi[MPa] | 76,500[527] | 77,600[535] | 79,250[546] | 78,350[540] |
| Elongation, % | 76 (8) | 71 (7) | 70 (5) | 70 (4.5) |
| Young's modulus, psi[GPa] | 620,100[4.28] | 611,600[4.22] | 641,100[4.42] | 635,400[4.38] |

Conclusions
Tiedown roughness properties

The dry and wet tiedown roughness properties for the Example 1, 2, and 3 sutures are reduced using a coating of polyester deposited on the surface of the suture as contrasted to the tiedown roughness values seen on the uncoated controls. As the level of coating is increased on the suture there is a proportionate decrease in the dry and wet tiedown roughness slide force. There is some variability in the dry and wet tiedown roughness data from one coating level to another possible due to the variability of the coating on the sutures.

The dry tiedown roughness properties of polypropylene are reduced by the polyester coating but the wet tiedown roughness properties are not reduced versus the dry and wet roughness properties of the uncoated polypropylene suture control.

Knot security evaluation

Dry and wet knot security tests were run for coated and uncoated Example 1, 2, and 4 sutures. There was insufficient quantity of the Example 3 suture material to run the knot security tests.

The dry and wet knot security properties of the coated Example 1 suture were not compromised by the coating. The coated Example 1 sutures had equal knot security properties when constructed to the uncoated suture control.

Polyester coated ethylene-propylene copolymer sutures required one extra throw to secure the knot as contrasted to the uncoated suture control. The extra throw to secure the knot was consistent in both the dry and wet knot security tests.

Polyester coated polypropylene sutures required one extra throw to secure the knot in the dry and wet knot security tests. The knot security data for this suture was variable possibly due to the variability of the coating on the sutures. The dry knot security appraised shows that the higher level of coating requires one extra knot for secuity whereas the wet knot security appraisal shows that the lower level of coating required one extra knot for security.

Knot and straight strength tensile properties

The overall appraisal of the knot strength and straight strength tensile properties indicates that the polyester coatings had no adverse effects on the tensile properties of the coated sutures as compared to their uncoated suture controls.

Visual appearance, Tactile smoothness and modulus properties

*Visual appearance*—The visual appearance of the polyester coated sutures are good.

*Tactile smoothness*—With increased levels of coating the tactile smoothness of coated Example 1, 2, and 4 sutures showed more drag than the uncoated suture controls. The tactile smoothness of coated Example 3 sutures at all coating levels was equal in smoothness when contrasted to the uncoated suture control.

*Modulus properties*—The Youngs Modulus of the coated sutures was not affected by the coating when contrasted to the uncoated suture controls.

*Microscopic appearance*—After tiedown, all the coated sutures showed slight evidence of flattened areas, some fibrils and waviness and slight evidence of flaked coating.

The overall appraisal of the coated Example 1, 2, and 3 sutures was good. The coating for polypropylene sutures did not perform as well on this suture as it did on the other sutures. There is slight evidence of coating variability on the sutures. This is felt to be the result of the dip coating/centrifuge method that was used to coat the cut bundle sutures. This can be overcome by applying the coating to the individual monofilament sutures by the Suspended Drop Coating Method as part of of the extrusion process prior to annealing.

**Claims**

1. A suture having improved tie-down roughness performance comprising a monofilament, the outer surface of which is coated with an effective amount of an aliphatic polyester with a number average molecular weight within the range of from 1000 to 15,000, the acid component of which has at least four carbon atoms.

2. The suture of claim 1 wherein the coating is present on the suture in an amount within the range of from 0.03 to 3 weight percent, based on suture weight.

3. The suture of claim 1 or claim 2 wherein said suture is made from isotactic polypropylene, ethylenepropylene copolymer, vinylidene fluoride - hexafluoropropylene copolymer, or poly(tetramethylene terephthalate co-2-octadecenylsuccinate).

4. The suture of any one of claims 1 to 3 wherein the aliphatic polyester is poly(1,4-butylene adipate).

**Patentansprüche**

1. Chirurgischer Faden mit verbesserter Rauheitseigenschaft beim Verknoten, umfassend ein Monofilament, dessen äußere Oberfläche mit einer wirksamen Menge eines aliphatischen Polyesters mit einem Zahlenmittel des Molekulargewichts im Bereich von 1000 bis 15,000 überzogen ist, dessen Säurekomponente wenigstens vier Kohlenstoffatome aufweist.

2. Chirurgischer Faden nach Anspruch 1, worin der überzug auf dem Faden in einer Menge im Bereich von 0,03 bis 3 Gewichts-%, bezogen auf das Fadengewicht, vorhanden ist.

3. Chirurgischer Faden nach Anspruch 1 oder 2, wobei der Faden aus isotaktischem Polypropylen, einem Ethylen-Propylen-Copolymer, einem Vinylidenfluorid - Hexafluorpropylen - Copolymer oder Poly(tetramethylenterephthalat - co - 2 - octadecenylsuccinat) besteht.

4. Chirurgischer Faden nach einem der Ansprüche 1 bis 3, worin der aliphatische Polyester Poly-(1,4-butylenadipat) ist.

**Revendications**

1. Suture ayant un comportement de ruguosité de fixation amélioré comprenant un monofilament, dont la surface extérieure est revêtue d'une quantité efficace d'un polyester aromatique ayant un poids moléculaire moyen en nombre dans un intervalle de 1 000 à 15 000, dont le composant acide a au moins 4 atomes de carbone.

2. Suture de la revendication 1, dans laquelle le revêtement est présent sur la suture en une quantité situé dans un intervalle allant de 0,03 à 3% en poids, sur la base du poids de la suture.

3. Suture de la revendication 1 ou de la revendication 2, dans laquelle ladite suture est faite de polypropylène isotactique, de copolymère éthylène-propylène, de copolymère fluorure de vinylidène-hexafluoropropylène ou de poly(tétraméthylène térephtalate co - 2 - octadécénylsuccinate).

4. Suture selon l'une quelconque des revendications 1 à 3, dans laquelle le polyester aliphatique est le poly(1,4 - butylène adipate).

FIG-1

FIG-2

# FIG-3

ROUGHNESS

FORCE

DISPLACEMENT

COATED SUTURE          UNCOATED SUTURE

# FIG-4

KNOT PULLED APART
IN INSTRON TESTER

KNOT TIED
ON MANDREL

TAIL

34

MANDREL

44

40

CUT

INSTRON
CLAMP

KNOT

36

38

42

32

SUTURE

INSTRON
CLAMP